# EUROPEAN PATENT APPLICATION

(11) **EP 3 929 280 A1**
(43) Date of publication of application: **29.12.2021**
(21) Application number: 20182747.4
(22) Date of filing: 26.06.2020
(51) Int. Cl.: C12N 5/071, A61K 35/407

(54) **PROCESS FOR PRODUCING LIVER CELLS**

(71) Applicant: Medizinische Hochschule Hannover, 30625 Hannover (DE)
(72) Inventor: BOCK, Michael, 30629 Hannover (DE); FEKETE-DRIMUSZ, Nora, 30171 Hannover (DE); VONDRAN, Florian Wolfgang Rudolph, 30625 Hannover (DE)
(74) Representative: Taruttis, Stefan Georg

(57) **Abstract**

The present invention relates to a process for producing liver cells, especially liver stem cells, which after injection into a mammal or in vitro form liver cells that are differentiated, especially differentiated into hepatocytes, into cholangiocytes, and preferably also into liver sinusoidal endothelial cells (LSEC) that can e.g. form blood sinusoidal capillaries. The invention is based on in vitro producing liver stem cells from a sample of liver tissue, cultivating the liver stem cells in vitro for an increase in cell number. It has been found that the liver stem cells that are produced by the process of the invention can be cultivated and increased in number while maintaining their capability to differentiate into liver cells, especially into hepatocytes, cholangiocytes, and preferably also into LSEC. Accordingly, the process of the invention is suitable for producing liver stem cells that are autologous for the originator of the sample of liver tissue.

## Description

The present invention relates to a process for producing liver cells, especially liver stem cells, which after injection into a mammal or in vitro form liver cells that are differentiated, especially differentiated into hepatocytes, into cholangiocytes, and preferably also into liver sinusoidal endothelial cells (LSEC) that can e.g. form blood sinusoidal capillaries.

The invention is based on in vitro producing liver stem cells from a sample of liver tissue, cultivating the liver stem cells in vitro for an increase in cell number. It has been found that the liver stem cells that are produced by the process of the invention can be cultivated and increased in number while maintaining their capability to differentiate into liver cells, especially into hepatocytes, cholangiocytes, and preferably also into LSEC. Accordingly, the process of the invention is suitable for producing liver stem cells that are autologous for the originator of the sample of liver tissue. The invention also provides liver cells, especially liver stem cells, for use in the treatment of liver defects, preferably autologous liver cells for use in the treatment of liver defects in the mammal from which the sample of liver tissue originates. Accordingly, the invention provides a method for treatment of liver defects by introducing the liver stem cells that are produced by a process according to the invention. Introducing the liver stem cells can be injection into a blood vessel connected to the liver, e.g. into the portal vein of the liver to be treated, or into the liver or a liver lobe itself, and especially in mice by intrasplenic injection.

Further, the invention provides experimental animals, e.g. mice or rats, that contain heterologous liver cells, e.g. human liver cells, preferably a liver consisting of human liver cells, and a process for generating such experimental animals. Further, the invention provides in vitro processes for analysing compounds, the processes comprising the step of providing liver cells that are produced according to the invention and contacting these liver cells with an agent to be analysed for its effect on liver cells. Therein, the liver cells can be liver stem cells or liver cells obtained by in vitro differentiating the liver stem cells, e.g. differentiated into hepatocytes, cholangiocytes, and/or LSEC.

### State of the art

Herrera et al, Stem Cells 24, 2840-2850 (2006), describe that by stringent conditions of liver cell cultures, human liver stem cells were isolated and in presence of hepatocyte growth factor and fibroblast growth factor 4 differentiated into mature hepatocytes. Hepatocytes were isolated by collagenase digestion of healthy liver tissue and mincing and pipetting. After two weeks cultivation, the large majority of hepatocytes were dead and after changing medium to α-minimum essential medium/endothelial cell basal medium-1 3:1, with supplements, surviving cells were cultured, and after another 3 weeks, individual attached cells were observed, then subcultured and expanded until confluence. These cells are described as being human liver stem cells that express markers of both mesenchymal stem cells (MSC) and immature liver cells.

Kim et al., J. Hepatol. 70, 97-107 (2019) describe that hepatocytes obtained by collagenase treatment from cancerous liver biopsies were cultivated overnight and then re-programmed during cultivation by adding small molecule synthetic compounds and HGF to generate hepatic progenitor cells, which had the potential to differentiate in vitro into functional hepatocytes and biliary epithelial cells.

Corbett and Duncan, Frontiers in Medicine, vol. 6, article 265 (2019) review hepatocytes generated from iPSC, wherein induced pluripotent stem cells (iPSC) could be generated from somatic cells by lentiviral transduction. These iPSC-derived hepatocytes are described as not having a fully mature phenotype of hepatocytes.

### Object of the invention

It is an object of the invention to provide an alternative process for producing liver cells, preferably having liver stem cell properties, that can be used to form hepatocytes, cholangiocytes, and preferably also LSEC, especially after introduction of the liver cells into a mammal for use in re-constituting defects of the liver tissue in vivo. Preferably, the process shall reproducibly produce the liver cells from a small liver tissue sample, and generate large numbers of the liver cells having liver stem cell properties, especially for use as autologous cells in the treatment of liver defects in the original donor of the liver tissue sample. A further object is to provide a process for in vitro producing liver cells, preferably aggregates of liver cells containing hepatocytes, cholangiocytes, and/or LSEC, which liver cells can be used in analytical in vitro processes. A further object is to provide an experimental animal, e.g. a rodent, the liver of which consists of human cells, and to provide a process for generating such experimental animals.

### Description of the invention

The invention achieves the objects by the features of the claims, especially by providing a process for producing liver cells, especially liver stem cells, from liver cells originating from a sample of liver tissue, preferably originating from a sample of human liver tissue, which sample can be fresh, maintained in culture medium, or frozen. In the process, the liver cells originating from a sample of liver tissue, which cells herein are also termed primary liver cells, are incubated in cell culture medium under cell culture conditions, preferably under static conditions, wherein the culture medium contains EGFL6, preferably human EGFL6 (hEGLF6), e.g. added to a concentration of 20-1000ng/mL medium. Generally, herein cell culture medium is suitable for cultivating live cells, and is also simply termed medium. It was found that presence of EGFL6 in the medium contacting the primary liver cells results in the production of liver cells that show characteristics of stem cells, which cells therefore herein are referred to as liver stem cells. In addition to stem cell characteristics, the liver stem cells also present markers that are indicative of hepatocytes, of cholangiocytes, and/or of LSEC.

According to present knowledge, the state of the original liver tissue, e.g. whether healthy, derived from young or aged, even deceased, male or female donors or very fatty or cirrhotic, does not have a significant influence, as liver stem cells could be generated from all clinical samples that were tested, both fresh and frozen, and no differences or aberrations were determined in liver stem cells produced from such samples.

Unexpectedly, presence of EGLF6 was found to result in production of liver stem cells irrespective of the state of the sample of liver tissue from which the primary liver cells were isolated. Primary liver cells can be isolated by collagenase treatment of a sample of liver tissue, preferably followed by mechanical shearing, e.g. passing of the collagenase treated tissue through a wide opening e.g. of a wide-bore pipette, and subsequent separation of single cells or of cell aggregates, e.g. by filtration and/or centrifugation, e.g. density gradient centrifugation.

The process comprises the steps of isolating liver cells from a sample of liver tissue, which preferably is human liver tissue, and incubating the cells, preferably under static conditions, in cell culture medium containing EGFL6, wherein the EGFL6 can be contained in the medium by being added to the medium, or the EGFL6 can be generated during the cultivation in the medium due to secretion of EGFL6 by liver stem cells. For generating EGFL6 by secretion of EGFL6 from liver stem cells, the primary liver cells include at least one, preferably more, polynuclear liver cells, as it has been observed that during cultivation smaller cells separate from polynuclear liver cells, which smaller cells have been found to be liver stem cells.

Human EGFL6 (human epidermal growth factor-like protein 6 isoform 1 precursor) comprises or consists of the amino acid sequence

MPLPWSLALPLLLSWVAGGFGNAASARHHGLLASARQPGVCHYGTKLACCYGWRR
NSKGVCEATCEPGCKFGECVGPNKCRCFPGYTGKTCSQDVNECGMKPRPCQHRCVN
THGSYKCFCLSGHMLMPDATCVNSRTCAMINCQYSCEDTEEGPQCLCPSSGLRLAPN
GRDCLDIDECASGKVICPYNRRCVNTFGSYYCKCHIGFELQYISGRYDCIDINECTMD
SHTCSHHANCFNTQGSFKCKCKQGYKGNGLRCSAIPENSVKEVLRAPGTIKDRIKKLL
AHKNSMKKKAKIKNVTPEPTRTPTPKVNLQPFNYEEIVSRGGNSHGGKKGNEEKMK
EGLEDEKREEKALKNDIEERSLRGDVFFPKVNEAGEFGLILVQRKALTSKLEHKDLNI
SVDCSFNHGICDWKQDREDDFDWNPADRDNAIGFYMAVPALAGHKKDIGRLKLLLP
DLQPQSNFCLLFDYRLAGDKVGKLRVFVKNSNNALAWEKTTSEDEKWKTGKIQLYQ
GTDATKSIIFEAERGKGKTGEIAVDGVLLVSGLCPDSLLSVDD (SEQ ID NO: 1).

Preferably, EGFL6, more preferably human EGFL6, is the expression product of cell culture, preferably of expression in mammalian cell culture.

In an embodiment, EGFL6 can be provided to the primary liver cells by cultivating them under static conditions in cell culture medium, which originally is devoid of EGFL6, and without addition of EGFL6, e.g. without addition of external EGFL6 or of a precursor of EGFL6 and without addition of an expression vector encoding EGFL6 or a precursor of EGFL6. It was found that under static conditions, the liver cells which originate from the polynuclear liver cells can produce and preferably secrete EGFL6 sufficient to produce liver stem cells. According to present observations, the presence of EGFL6, added to the medium or secreted by cells, seems to initiate a of primary liver cells into liver stem cells. The static culture of the primary liver cells preferably is for at least 4 days, at least 5 days, more preferably for at least 6 days, at least 7 days or at least 8 days, more preferably for at least 14 days, even more preferably for at least 21 days, without movement and in the same medium. Herein, the static culture defines conditions of cultivation without any movement of medium in relation to the culture vessel or in relation to the cells, e.g. without any movement of culture vessel, without any movement of medium, leaving the medium without manipulation. The static culture provides an artificial environment for the primary liver cells, which is in stark contrast to the continuous movement generated by the continuous perfusion conditions present in natural liver tissue. Preferably, especially in this embodiment, primary liver cells are seeded at a density of at least 100 cells per mm² bottom surface of the culture vessel, more preferably at a density of at least 1000 or at least 2,000 cells per mm² bottom surface of the culture vessel. Correspondingly, in an embodiment, the process comprises cultivating primary liver cells in culture medium, wherein the culture medium is devoid of EGFL6 and devoid of a precursor of EGFL6 and devoid of an added expression vector encoding EGFL6 or a precursor of EGFL6, wherein the primary liver cells comprise at least one or more polynuclear liver cells. In the alternative to or in addition to the primary cells containing at least one polynuclear cell, primary cells can be selected that contain at least one cell expressing EGFL6. EGFL6 expressing cells can be detected by immunologic detection, e.g. using FACS and a labelled antibody that is specific for EGFL6. As EGFL6 has been found to be secreted, detection of primary liver cells that contain at least one cell expressing EGFL6 can be by taking an aliquot from a preparation of primary liver cells, determining, e.g. after permeabilization of the cells, the presence of EGFL6-positive cells, and using for cultivation the remaining portion of the primary liver cells. The medium generally, and especially in this embodiment, as a serum component only contains human serum, therefore the medium being devoid of fetal calf serum or serum replacement agents.

Preferably, a lower oxygen concentration and/or higher CO₂ concentration are applied during a part of or during the entire cultivation period, e.g. by reducing the gas exchange rate with a 5% CO₂ atmosphere, e.g. by closing the gas vents of static culture dishes to leave an exchange cross-section of only 1/10^{th} to 1/20^{th}, e.g. of standard culture plates having the gas vent between the rim of the plate and the lid. Accordingly, at least during the incubation of primary liver cells, the medium is in contact with a gas atmosphere having a lower oxygen concentration than the standard atmosphere and/or a higher CO₂ concentration than 5 Vol.-% during the entire cultivation period.

Preferably, the primary liver cells contain at least one polynuclear liver cell, and optionally, the process comprises the step of analysing the primary liver cells for presence of polynuclear cells and cultivating only primary liver cells that contain at least one polynuclear cell. The step of analysing can e.g. be by microscopic analysis, e.g. using phase contrast light microscopy. Herein, a polynuclear cell contains at least two nuclei. Polynuclear liver cells that are present among the primary liver cells that are cultivated were found to give rise to smaller cells which are liver stem cells and to produce EGFL6 in their vicinity, resulting in the production of liver stem cells. Accordingly, in embodiments in which the medium contains no added EGFL6, primary liver cells that do not contain a polynuclear cell preferably are not cultivated, but only primary liver cells that contain at least one polynuclear liver cell are cultivated for producing liver stem cells. Generally, especially in embodiments in which the medium contains no added EGFL6, polynuclear liver cells are enriched from the primary liver cells to produce a primary liver cell fraction that is enriched for polynuclear cells, and cultivating this cell fraction in medium containing no added EGFL6. Optionally, the medium is replaced by fresh medium that is devoid of added EGFL6, e.g. when passaging the cells, preferably using static culture conditions, optionally in combination with increased CO₂ concentration and/or reduced O₂ concentration in the gas phase.

In the process for producing liver stem cells, it is preferred that the medium for producing liver stem cells contains EGFL6 in all passages, as it has been found that in the presence of EGFL6 in the medium, the liver stem cells can be passaged at least 30 times, preferably at least 40 times while the cells maintain their liver stem cell characteristics and in each passage proliferate, i.e. do not show signs of aging or apoptosis. During the passages, liver stem cells can be allowed to proliferate up to confluence or close to confluence, e.g. up to 95% or up to 90% of confluence. As the presence of EGFL6 in the medium was found to support proliferation of the liver stem cells without changes, especially without genetic changes occurring in the liver stem cells, the cultivation in medium containing EGFL6 can be cultivation in suspension or adherence culture for a time that is equivalent to at least 30, preferably at least 40 passages of cell culture in static or agitated culture plates, which are e.g. passages after reaching 90% or 95% confluence.

Liver stem cells produced by the process have been found to differentiate in vitro and in vivo into hepatocytes, into cholangiocytes, and preferably into LSEC. For differentiation in vitro, the liver stem cells can be cultivated in medium that is devoid of EGFL6, e.g. by exchanging the medium for fresh medium that is devoid of EGFL6, e.g. by adding an antibody specific for EGFL6 in order to mask EGFL6. An antibody specific for EGFL6 preferably binds to the C-terminal portion of EGFL6, and the antibody can be a monoclonal, preferably a polyclonal antibody specific for EGFL6. Further, differentiation can be induced by contacting the liver stem cells in vitro with a differentiation factor, which is e.g. added to the medium.

As the process allows for the production of liver stem cells without a limitation having occurred so far, liver stem cells can be produced by cultivation essentially endlessly, providing liver stem cells that are genetically identical and stable, especially in respect of their natural genes.

As the liver stem cells can subsequently be differentiated, the differentiated cells can be produced essentially without limitation, each cell being genetically identical and stable, especially in respect of their natural genes. The process therefore has the general advantage of producing liver stem cells that are genetically identical, and differentiated cells that are genetically identical, without limitation in number, and preferably without the need for preservation of cells. The liver stem cells and differentiated cells produced by the process are therefore suitable for processes analysing the effect of agents onto liver cells, as results can be compared and repeatedly generated with the same kind of cells, e.g. without inherent cell-to-cell variation.

Generally, the liver stem cells produced by the process of the invention have the advantage that by the process, no transduction and no transformation towards propagative properties and immortality is introduced into the cells. Accordingly, the liver stem cells as well as cells generated from these liver stem cells by differentiation do not contain tumour cells. In contrast, for example, cell lines generated from original tumour tissue can be immortal under cultivation conditions because they are transformed to tumour cells, as can be seen for most cell lines. As a further advantage, the process and accordingly the liver stem cells produced by the process, do not comprise genetic manipulations for effecting the production of liver stem cells from the primary liver cells, e.g. the process and the liver stem cells are devoid of nucleic acid constructs comprising genes for reprogramming cells, e.g. devoid of introduced nucleic acid constructs suitable for induction of stem cell properties.

In an embodiment, the invention provides a process for analysing the effects of agents onto liver cells, which are liver stem cells produced by the process of the invention, with optional in vitro differentiation of liver stem cells, e.g. differentiated into hepatocytes, cholangiocytes and/or LSEC.

Further optionally, the liver stem cells, with optional differentiation e.g. into hepatocytes, cholangiocytes and/or LSEC, can be cultivated to produce cell aggregates that contain at least two, preferably all three of hepatocytes, cholangiocytes and LSEC. Processes for analysing the effects of agents onto liver cells can be performed on these aggregates of liver cells. Differentiation of liver stem cells can in vitro be obtained by adding known differentiation factors, e.g. hepatocyte maturation factors, Oncostatin M/dexamethasone for differentiation into hepatocytes, culture with cholangiocyte maturation factors, contained in Matrigel, for differentiation into cholangiocytes, and/or LSEC maturation factors, e.g. VEGF and/or nitric oxide, preferably under enhanced hypoxia/hypercapnia culture conditions for differentiation into LSEC, or a combination of at least two of these.

For differentiation in vivo, the liver stem cells can be injected into a mammal having defective sites in its liver tissue, and it was found that following injection the liver stem cells settle in defective sites of liver tissue and form, or mature into, hepatocytes, cholangiocytes, and preferably LSEC, generating viable and functionally active liver tissue. The mammal can be an experimental animal or a human patient. Preferably the liver stem cells are injected into the mammal in close proximity of the defective site of liver tissue, e.g. intrasplenically or into the portal vein. It has been found in mouse experiments that the liver stem cells after injection into the blood stream or into the spleen form liver tissue essentially only at the liver tissue site, e.g. within liver tissue or within defective liver tissue, and do not generate liver tissue at other sites of the mammal. Accordingly, the liver stem cells of the invention are suitable for use in the treatment of liver defects, e.g. in humans, wherein preferably the primary liver cells originate from a sample of liver tissue of the later recipient of the liver stem cells.

Accordingly, the liver stem cells that are produced from primary liver cells of a sample of autologous liver tissue are autologous and therefore immunologically compatible with the later recipient. It was found that the process for producing liver stem cells does not change the immunologic properties compared to the original primary liver cells.

In an embodiment, the liver stem cells, which preferably are generated from human primary liver cells, are injected into an experimental animal having defective liver tissue, in order to generate an experimental animal having a liver that comprises, preferably consists of human liver cells. Such experimental animals are suitable for processes analysing the effect of agents onto the liver, as the liver of such animals comprises or consists of human liver cells. An exemplary experimental animal has a defective immune system, e.g. a Rag2-/- and γc-/- mouse (RAG mouse), or a FAH-/- and Rag2-/- and γc-/- mouse (FRG mouse), in order to prevent rejection of the human cells. The defective liver can be generated by chemical, herein by administration of 0.5 mL CCl₄/kg bodyweight 1 day prior to injection of the liver stem cells, or mechanical means, or the original mouse can be genetically defective thus exhibiting chronic liver damage.

Characteristic stem cell markers found in the liver stem cells produced by the process comprise or consist of the combination of NCAM-1, PKM1/M2, SALL4, SOX17, MCAM, TERT, CD90, preferably the combination of KRT18, KRT19, TERT, HNF4A, FOXA2, and LYVE1, and optionally including EGFL6; more preferred the combination of KRT8, KRT18 CYP3A4, KRT7, KRT19, OPN, CFTR, TERT, EPCAM, HNF4A, FOXA2, CEBPA, CEBPB, LYVVE1, VWF, and optionally including EGFL6; even more preferred the combination of KRT8, KRT18 CYP3A4, ALB, TDO, KRT7, KRT19, OPN, CFTR, TERT, EPCAM, PKM, SALL4, NCAM1, THY1, HNF4A, FOXA2, CEBPA, CEBPB, LYVVE1, VWF, KDR, SELE, F8, and optionally including EGFL6.

Generally, markers of the liver stem cells can be determined by fluorescence assisted cell sorting (FACS) using labelled antibodies having specificity for the markers. Characteristic markers of the liver stem cells comprise markers that are indicative of hepatocytes, comprising ALB (albumin), TDO, HNF4a, CYP3a4, KRT8, KRT18 and FAH, as well as markers that are indicative of cholangiocytes, comprising AE2, SCTR, OPN, KRT7, KRT19, CFTR, GGT, as well as markers that are indicative of LSEC, comprising VWF, F8, CD32b, LYVE-1, SEL-E, KDR, FLT4.

Further, the liver stem cells have been found to have the signalling receptors TNFRSF1A, Notch2, Patchedl, Catenin-b, YEP1, RYK, LGR5, and the transcription factors C/EBPa, C/EBPb, FOXA2, as well structural factors E-Cad, N-Cad, EpCAM, TWF1.

This shows that the liver stem cells have characteristic markers both for being stem cells and for being hepatocytes and cholangiocytes, and preferably also LSEC.
Optionally, the process can provide the liver stem cells which in addition are genetically manipulated, e.g. by introduction of a nucleic acid construct into the liver stem cells, e.g. during the cultivation in medium containing EGFL6. Nucleic acid constructs can be introduced by viral transduction of the cells, by transfecting or injecting nucleic acid constructs into the cells or by another process known for genetically manipulating mammalian cells. In this embodiment, the process can provide liver stem cells that are genetically manipulated to contain a functionally active gene of a gene that is defective in the primary liver cells obtained from the later recipient. In this embodiment, the process has the advantage of providing genetically manipulated liver stem cells which during the production can be analysed and controlled, as the process for production is an in vitro process. Therefore, the process is free from genetic manipulation procedures involving in vivo steps of introducing nucleic acid constructs into liver cells. In this embodiment, the process provides liver stem cells that are genetically manipulated to contain a functionally active gene for use in the treatment of a genetic defect, e.g. in the liver. Therein, the genetic defect can be a dysfunction of a gene and the functionally active gene is a functional copy of the gene, or the genetic defect can be a detrimental overactivity of a gene and the functionally active gene can be a functional copy of the gene having its normal activity of a healthy status.

The invention is now described by way of examples and with reference to the figures, which show in
- Fig. 1 a) to f) light micrographs of an exemplary culture of primary human liver cells developing into liver stem cells,
- Fig. 2 graphical representations of analytical results for specific markers of liver stem cells produced according to the invention, and in
- Fig. 3 a) to c) histological analyses of liver tissue developed from human liver stem cells in mice.

### Example 1: Process for producing liver stem cells from primary human liver cells

Primary liver cells were prepared from human liver tissue that was received from liver surgery. Prior analysis had shown that the liver tissue was no tumour tissue. The liver tissue was digested by collagenase treatment using 0.05% collagenase P in pre-warmed digestion buffer at 37°C for 15-20min min, followed by further mechanical disruption and pouring of the emerging cell suspension through a gauze-lined funnel and centrifuged (50 x g, 5 min, 4°C). The isolated cells, now regarded as primary liver cells, were seeded at a density of at least 400 000 cells in culture dishes (2.2cm diameter) that were non-coated plastic, optionally coated with Collagen I, in cell culture medium. The medium could be Williams E, RPMI 1640, DMEM(:F12), Promocell Hepatocyte Growth Medium, preferably hepatocyte maintenance medium Lonza, e.g. HCM. Generally preferable, the medium contained human serum, e.g. 1-10 Vol.%.

Cultivation was static at 37°C in a 5% CO₂ atmosphere, preferably the circumferential gas vents of these static culture dishes were closed by adhesive tape to leave an exchange cross-section of only 1/10^{th} to 1/20^{th} of the total circumference. Cultivation was for 4 to 8 days without movement before removing the dishes from the incubator to check cells under a microscope. The medium was not exchanged during the initial 29 days of the culture.

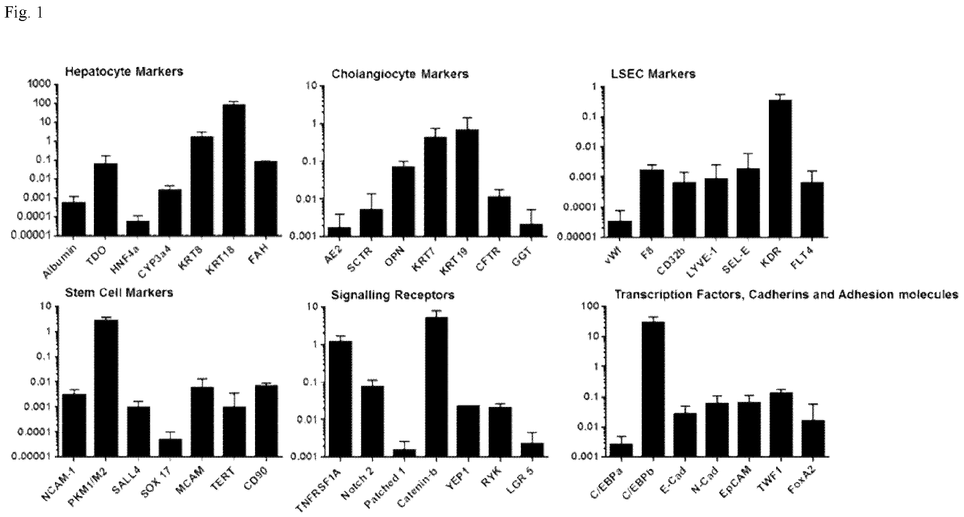

Fig. 1 shows light microscopic pictures (size bar is 250 µm) of the same section of one exemplary culture dish, a) at day 14, b) at day 17, c) at day 28, d) at day 23, e) at day 26, and f) at day 29 after seeding. This shows the albeit slow occurrence of optically differing cell clusters. Cells of these clusters have been characterized later as being EGFL6⁺-liver stem cells. These liver stem cells could be propagated, e.g. when reaching 90% confluence or more, with trypsination and passaging to further dishes, using the same fresh medium, again under static culture conditions.

### Example 2: Process for producing liver stem cells from primary human liver cells

In accordance with the preferred embodiment, primary liver cells that were isolated from a human liver tissue sample were incubated in medium containing added human EGFL6 (obtained from Sino Biological), e.g. at a concentration of 200ng/ml.

Cultivation of primary liver cells in medium containing EGFL6 resulted in the production of liver stem cells both from polynuclear liver cells, and from mononuclear liver cells. The occurrence of clusters of liver stem cells in static culture therefore was possible for all primary liver cells, and it was more efficient than in the process using cultivation without added EGFL6.

Passaging of liver stem cells, e.g. when reaching up to 90% confluence, to-date is at least 40 passages, resulting in an increase 2-10fold/passage of the number of liver stem cells.

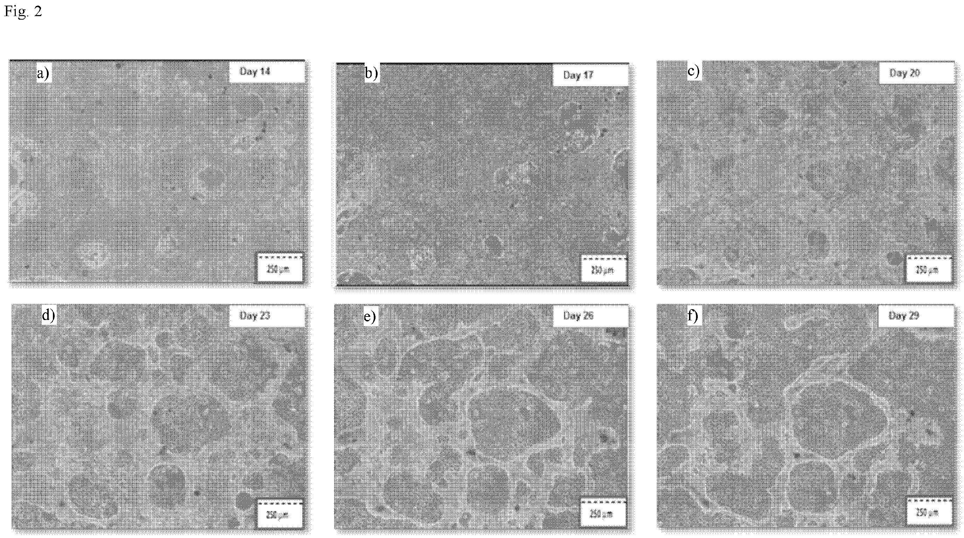

The liver stem cells produced by the process were analysed by reverse-transcription quantitative PCR (RT-qPCR). In short, total mRNA was isolated from cultivated liver stem cells using RNeasy Plus micro Kit (obtained from Qiagen), and mRNA was analysed by RT-qPCR using primer pairs specific for markers as indicated in Fig. 2, which shows the results. In Fig. 2, mean quantities of the specific amplificates and standard deviations are indicated for liver stem cells that were generated from liver tissue samples obtained from four different patients, all non-cancer patients.

The result shows that the EGFL6-expressing liver stem cells produced by the process of the invention express markers for hepatocytes, for cholangiocytes and for LSEC, which in their combination and with expression of signalling receptors are also regarded as stem cells of endodermal origin in developmental terms. In detail, for liver stem cells according to the invention robust transcript levels for ALB, TDO, HNF4A, CYP3A4, KRT8, KRT18 and FAH indicated hepatocytes; SLC4A2 (AE2), SCTR, OPN, KRT7, KRT19, CFTR, GGT indicated cholangiocytes; VWF, F8, FCGR2B (CD32b), LYVE1, SELE, KDR, FLT4 indicated LSEC; NCAM1, PKM, SALL4, SOX17, MCAM, TERT, THY1 (CD90) indicated stem cells; TNFRSF1A, NOTCH2, PTCH1, CTNNB1 (b-Catenin), YEP1, RYK, LGR5 are signalling receptors indicating response potential for the particular signalling pathways; and CEBPA, CEBPB, FOXA2, together with HNF4A are transcription factors also indicating endodermal origin; CDH1 (E-Cad), CDH2 (N-Cad), EPCAM, TWF1, are cadherins and adhesion molecules indicating both epithelial as well as mesenchymal cell potential.

### Example 3: Process for generating an experimental animal containing liver tissue generated from human liver stem cells

The process for generating an experimental animal containing human liver tissue also exemplifies the use of the liver stem cells for treatment of liver defects in human patients. For use in human patients, the liver stem cells are preferably generated from a liver tissue sample obtained from the patient, who is the later recipient of the liver stem cells, and therefore the liver stem cells are autologous cells that are immunologically compatible to the patient.

Human liver stem cells produced according to Example 2 and as an example for genetic in vitro manipulation were transduced with a lentiviral nucleic acid construct encoding GFP under the control of a SFFV promoter.

These genetically modified human liver stem cells were introduced by injection into the liver portal vein of two strains of immune deficient mice, Rag2-/- and γc-/- mouse (RAG mouse), or a FAH-/- and Rag2-/- and γc-/- mouse (FRG mouse), 5 mice each. FAH^{-/-} mice had a chronically defective liver due to withdrawal of the drug NTBC after cell transplantation. After collecting the human liver stem cells from the culture plate, they were resuspended in PBS, and then were intrasplenically injected at a quantity of ca. 500 000 cells/mouse, using a wide gauge needle. All animal care regulations and anaesthesia were applied.

The RAG mice were kept with standard laboratory mouse diet. FRG mice were kept with standard laboratory diet but drinking water was supplemented with NTBC before injection of liver stem cells and drinking water without NTBC after injection of liver stem cells, in order to induce chronic cell damage in the endogenous murine liver and, after injection of the human liver stem cells, promoting growth of the injected human cells.

After about 90 days, mice were killed and fully examined. It was found that no liver cells were present outside of the normal location of liver, and the liver was partially or completely made up of human cells.

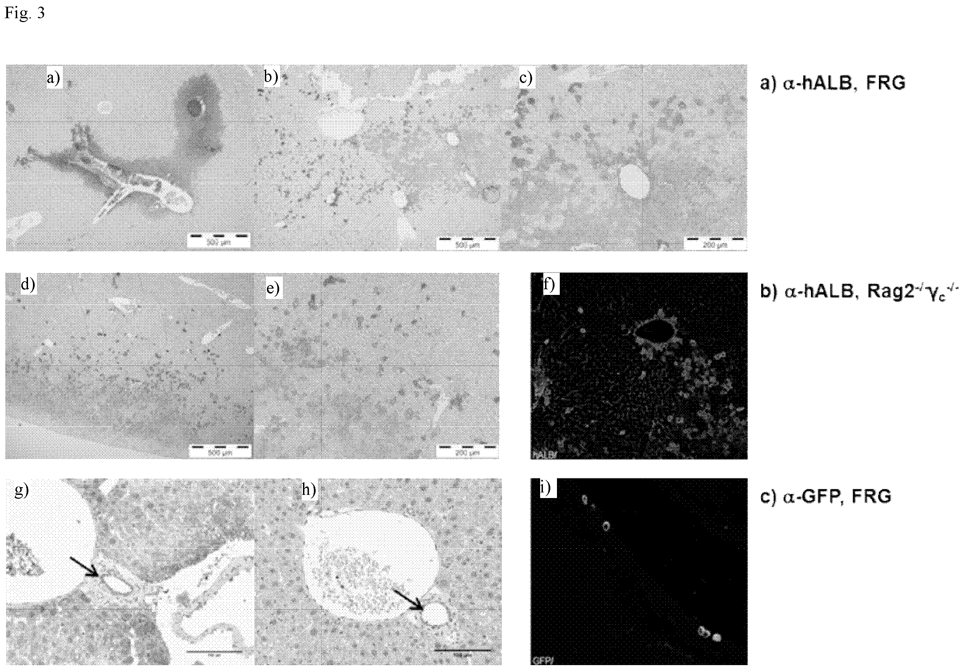

Fig. 3 a) to i) shows micrographs of mouse liver sections, in Fig. 3 a), b) and c) of an exemplary FRG mouse and in Fig. 3 d), e) and f) for an exemplary Rag2-/- γc-/- mouse. Immune histochemical staining in a), d) and g) was by HRP/DAPI, immune fluorescence staining in b), e) and h) was by FITC. Immune staining for human Albumin using an antihuman albumin antibody (a-hALB, obtained from Bethyl Laboratories) is shown in c) for a FRG mouse, and in f) for a Rag2-/- γc-/- mouse. Fig. 3 i) shows immune staining for GFP using an anti-GFP antibody (a-GFP, obtained from Abcam) in the liver of a FRG mouse.

These results show that the human liver stem cells made up at least part of the liver tissue in the mouse, and specifically, the human liver stem cells generated non-hepatocytic LSEC-networks as indicated by staining for human Albumin (hALB-positive), indicating LSEC originating from the human liver stem cells. The liver sections of the FRG mouse in immune staining for GFP indicate whole bile ducts generated from the human liver stem cells. The immune staining via HRP/DAPI immunohistochemistry shows the lining of a bile duct by individual human cholangiocytes generated from the human liver stem cells.

## Claims

1. Process for producing liver cells, comprising isolating liver cells from a sample of liver tissue to generate primary liver cells and incubating the primary liver cells in cell culture medium containing EGFL6 in order to produce liver cells which are liver stem cells.

2. Process according to claim 1, **characterized in that** the EGFL6 is contained in the cell culture medium by addition of EGFL6 to the cell culture medium.

3. Process according to claim 2, **characterized in that** the primary liver cells are not controlled to contain polynuclear liver cells or are devoid of polynuclear liver cells.

4. Process according to claim 1, **characterized in that** the primary liver cells are controlled to contain at least one polynuclear liver cell, that the cell culture medium initially is devoid of added EGFL6, and that incubating is under static conditions for at least 14 days in contact with a gas atmosphere having a lower oxygen concentration than the standard atmosphere and/or a higher CO₂ concentration than 5 Vol.-% during the entire cultivation period.

5. Process according to one of claims 1 and 4, **characterized in that** the primary liver cells are controlled to contain at least one cell that expresses EGFL6.

6. Process according to one of the preceding claims, **characterized in that** the sample of liver tissue is a sample of human liver tissue.

7. Process according to one of the preceding claims, **characterized in that** the liver stem cells are in vitro contacted with at least one differentiation factor initiating differentiation into hepatocytes, into cholangiocytes, and/or into liver sinusoidal endothelial cells (LSEC).

8. Process according to one of the preceding claims, **characterized in that** the liver stem cells are contacted with an agent in a process for analysing the effect of the agent onto liver cells.

9. Process according to one of the preceding claims, **characterized in that** the liver stem cells are cultivated for a time that is equivalent to at least 30 passages in static cell culture plates.

10. Process according to one of the preceding claims, **characterized in that** the liver stem cells are genetically manipulated.

11. Process according to one of the preceding claims, **characterized in that** the liver stem cells are injected into a non-human mammal having a defective liver for producing a non-human mammal having a liver that is in part or completely comprised of human liver cells.

12. Liver stem cell, obtainable by a process according to one of the preceding claims, **characterized in that** the cell express the combination of markers EGFL6, KRT18, KRT19, TERT, HNF4A, FOXA2, LYVE1 and optionally including EGFL6.

13. Liver stem cell according claim 12 for use as a medicament in the treatment of liver defects.

14. Non-human mammal for use as an experimental animal, **characterized by** having a liver that at least in part is comprised of human liver cells.

15. Process for analysing compounds, the processes comprising the step of providing liver cells that are produced according to one of claims 1 to 11 or cells according to claim 12, and contacting these liver cells with an agent to be analysed for its effect on liver cells.


SEQUENCE LISTING

<110> Medizinische Hochschule Hannover

<120> Process

<130> M1093EP

<160> 1

<170> BiSSAP 1.3.6

<210> 1
<211> 553
<212> PRT
<213> Homo sapiens

<220>
<223> hEGFL6

<400> 1
Met Pro Leu Pro Trp Ser Leu Ala Leu Pro Leu Leu Leu Ser Trp Val
1               5                   10                  15
Ala Gly Gly Phe Gly Asn Ala Ala Ser Ala Arg His His Gly Leu Leu
            20                  25                  30
Ala Ser Ala Arg Gln Pro Gly Val Cys His Tyr Gly Thr Lys Leu Ala
        35                  40                  45
Cys Cys Tyr Gly Trp Arg Arg Asn Ser Lys Gly Val Cys Glu Ala Thr
    50                  55                  60
Cys Glu Pro Gly Cys Lys Phe Gly Glu Cys Val Gly Pro Asn Lys Cys
65                  70                  75                  80
Arg Cys Phe Pro Gly Tyr Thr Gly Lys Thr Cys Ser Gln Asp Val Asn
                85                  90                  95
Glu Cys Gly Met Lys Pro Arg Pro Cys Gln His Arg Cys Val Asn Thr
            100                 105                 110
His Gly Ser Tyr Lys Cys Phe Cys Leu Ser Gly His Met Leu Met Pro
        115                 120                 125
Asp Ala Thr Cys Val Asn Ser Arg Thr Cys Ala Met Ile Asn Cys Gln
    130                 135                 140
Tyr Ser Cys Glu Asp Thr Glu Glu Gly Pro Gln Cys Leu Cys Pro Ser
145                 150                 155                 160
Ser Gly Leu Arg Leu Ala Pro Asn Gly Arg Asp Cys Leu Asp Ile Asp
                165                 170                 175
Glu Cys Ala Ser Gly Lys Val Ile Cys Pro Tyr Asn Arg Arg Cys Val
            180                 185                 190
Asn Thr Phe Gly Ser Tyr Tyr Cys Lys Cys His Ile Gly Phe Glu Leu
        195                 200                 205
Gln Tyr Ile Ser Gly Arg Tyr Asp Cys Ile Asp Ile Asn Glu Cys Thr
    210                 215                 220
Met Asp Ser His Thr Cys Ser His His Ala Asn Cys Phe Asn Thr Gln
225                 230                 235                 240
Gly Ser Phe Lys Cys Lys Cys Lys Gln Gly Tyr Lys Gly Asn Gly Leu
                245                 250                 255
Arg Cys Ser Ala Ile Pro Glu Asn Ser Val Lys Glu Val Leu Arg Ala
            260                 265                 270
Pro Gly Thr Ile Lys Asp Arg Ile Lys Lys Leu Leu Ala His Lys Asn
        275                 280                 285
Ser Met Lys Lys Lys Ala Lys Ile Lys Asn Val Thr Pro Glu Pro Thr
    290                 295                 300
Arg Thr Pro Thr Pro Lys Val Asn Leu Gln Pro Phe Asn Tyr Glu Glu
305                 310                 315                 320
Ile Val Ser Arg Gly Gly Asn Ser His Gly Gly Lys Lys Gly Asn Glu

325                 330                 335
Glu Lys Met Lys Glu Gly Leu Glu Asp Glu Lys Arg Glu Glu Lys Ala
            340                 345                 350
Leu Lys Asn Asp Ile Glu Glu Arg Ser Leu Arg Gly Asp Val Phe Phe
        355                 360                 365
Pro Lys Val Asn Glu Ala Gly Glu Phe Gly Leu Ile Leu Val Gln Arg
    370                 375                 380
Lys Ala Leu Thr Ser Lys Leu Glu His Lys Asp Leu Asn Ile Ser Val
385                 390                 395                 400
Asp Cys Ser Phe Asn His Gly Ile Cys Asp Trp Lys Gln Asp Arg Glu
                405                 410                 415
Asp Asp Phe Asp Trp Asn Pro Ala Asp Arg Asp Asn Ala Ile Gly Phe
            420                 425                 430
Tyr Met Ala Val Pro Ala Leu Ala Gly His Lys Lys Asp Ile Gly Arg
        435                 440                 445
Leu Lys Leu Leu Leu Pro Asp Leu Gln Pro Gln Ser Asn Phe Cys Leu
    450                 455                 460
Leu Phe Asp Tyr Arg Leu Ala Gly Asp Lys Val Gly Lys Leu Arg Val
465                 470                 475                 480
Phe Val Lys Asn Ser Asn Asn Ala Leu Ala Trp Glu Lys Thr Thr Ser
                485                 490                 495
Glu Asp Glu Lys Trp Lys Thr Gly Lys Ile Gln Leu Tyr Gln Gly Thr
            500                 505                 510
Asp Ala Thr Lys Ser Ile Ile Phe Glu Ala Glu Arg Gly Lys Gly Lys
        515                 520                 525
Thr Gly Glu Ile Ala Val Asp Gly Val Leu Leu Val Ser Gly Leu Cys
    530                 535                 540
Pro Asp Ser Leu Leu Ser Val Asp Asp
545                 550